# FASCICULE DE BREVET EUROPEEN

(11) **EP 3 240 922 B1**
(45) Date de publication et mention de la délivrance du brevet: **07.08.2019**
(21) Numéro de dépôt: 15830820.5
(22) Date de dépôt: 21.12.2015
(51) Int. Cl.: D04H 1/4218, D04H 1/4226, D04H 1/58, G01N 33/36, B29C 67/24

(54) **PROCÉDÉ DE MESURE À L'INTÉRIEUR D'UN MATELAS DE FIBRES MINÉRALES OU VÉGÉTALES**
VERFAHREN ZUR MESSUNG INNERHALB EINER MATTE AUS MINERAL- ODER PFLANZENFASERN
METHOD FOR MEASURING INSIDE A BATT OF MINERAL OR PLANT FIBRES

(30) Priorité: 29.12.2014 FR 1463391
(43) Date de publication de la demande: 08.11.2017
(73) Titulaire: Saint-Gobain Isover, 92400 Courbevoie (FR)
(72) Inventeur: PELINI, Claire, 49000 Angers (FR); ZOWADA, Artur, 44100 Gliwice (PL); ASENSIO BAZTERRA, Francisco Javier, 31006 Pamplona (ES)
(74) Mandataire: Saint-Gobain Recherche
(86) Numéro de dépôt international: PCT/FR2015/053685
(87) Numéro de publication internationale: WO 2016/108006

(56) Documents cités:
- WO-A1-84/01430
- WO-A1-2007/107022
- WO-A1-2011/046863
- WO-A1-2013/015961
- WO-A1-2014/020265
- DE-A1- 19 902 759
- DE-A1-102010 038 817
- FR-A1- 2 984 371
- JP-A- 2007 139 272
- US-A1- 2006 009 569

## Description

L'invention se rapporte au domaine des procédés de mesure à l'intérieur d'un matelas continu de fibres minérales ou végétales, en particulier de laine minérale, de type laine de verre ou de roche, et un liant. Ces matelas sont destinés à être découpés pour former ensuite par exemple des panneaux ou rouleaux d'isolation thermique et/ou acoustique.

La fabrication de tels matelas de fibres isolantes comprend primairement le fibrage et le dépôt de fibres sur un convoyeur ou transporteur mobile perforé. L'amas de fibres néoformé est plaqué sur le convoyeur à l'aide de caissons d'aspiration agencés sous le transporteur sur lequel elles sont déposées. Lors du fibrage, un liant est pulvérisé à l'état de solution ou suspension dans un liquide volatil tel que l'eau sur les fibres étirées, ce liant ayant des propriétés d'adhésivité et comprenant d'habitude une matière durcissable à chaud, comme une résine thermodurcissable.

La couche primaire de fibres relativement lâches sur le convoyeur collecteur est ensuite transférée à un dispositif de chauffage appelé communément dans le domaine en cause étuve de réticulation. Le matelas de fibre traverse l'étuve sur toute sa longueur, grâce à des convoyeurs perforés supplémentaires. Ce sont fréquemment deux bandes sans fin en regard l'un de l'autre et espacés d'une distance ajustée pour déterminer l'épaisseur du matelas qui se forme. Chaque bande des convoyeurs est par ailleurs constituée de palettes formant des grilles articulées entre elles et perforées pour être perméables à l'air et aux autres gaz issus du chauffage du matelas. Un tel matelas présente ainsi une densité plus ou moins importante en fonction du degré de la compression exercée par les deux convoyeurs dans l'étuve.

Pendant son passage dans l'étuve, le matelas est simultanément asséché et soumis à un traitement thermique spécifique qui provoque la polymérisation (ou « durcissement ») de la résine thermodurcissable du liant présent à la surface des fibres.

Le mode opératoire utilisé pour provoquer le durcissement du liant consiste à faire passer de l'air chauffé dans le matelas, de telle façon que le liant présent dans toute l'épaisseur du matelas soit porté progressivement à une température supérieure à sa température de durcissement. A cette fin, l'étuve de réticulation est composée d'une enceinte constituant une chambre fermée autour du matelas, dans laquelle sont disposés une série de caissons alimentés en air chaud issu de brûleurs et mis en circulation par des ventilateurs. Chaque caisson définit ainsi une zone indépendante de chauffage, dans laquelle sont réglées des conditions spécifiques de chauffage. Les caissons sont séparés par des parois présentant des ouvertures pour le matelas et les convoyeurs supérieurs et inférieurs. L'utilisation d'une pluralité de caissons permet ainsi une élévation graduée de la température du matelas tout au long de sa traversée de l'étuve et évite l'apparition de points chauds dus à un chauffage localement trop important ou alternativement la présence au sein du matelas de zones dans lesquelles le liant n'aurait pas été entièrement polymérisé. Une étuve utilisée dans le procédé de fabrication de laine minérale comprend ainsi très couramment une multitude de caissons (par exemple entre 3 et 10), ainsi que des moyens connus permettant d'établir des conditions thermiques variables au sein de chaque caisson.

A l'heure actuelle, l'utilisation de nouveaux liants alternatifs, en remplacement des résines formo-phénoliques, rend le contrôle des conditions du procédé de cuisson du matelas de fibres dans une étuve classique telle que décrite précédemment plus difficile. De tels liants, généralement dépourvus de formaldéhyde, parfois qualifiés de « liants verts », notamment lorsqu'ils sont au moins partiellement issus d'une base de matière première renouvelable, en particulier végétale, notamment du type à base de sucres hydrogénés ou non, par exemple tel que décrit dans la les demandes WO 2009/080938 et WO 2010/029266, nécessitent le plus souvent une très bonne régulation des températures de cuisson pour atteindre l'état thermodurci, la gamme de température de cuisson étant plus étroite. Tout particulièrement le liant doit être soumis à une température comprise entre un minimum pour achever son durcissement et un maximum au-delà duquel il se dégrade rapidement, ce qui se traduit au final par des propriétés mécaniques dégradées du produit final, même après son installation. La différence entre le minimum et le maximum, en fonction du type de liant vert, peut être de l'ordre de seulement 20°C, voire moins. Le contrôle de la température dans toute l'épaisseur et toute la largeur du matelas de fibres nécessite donc de nouvelles techniques et en particulier des changements dans la conception même des étuves. Un exemple d'étuve est décrit dans la demande FR 2 984 371.

Un but de l'invention est d'avoir un procédé de fabrication permettant d'obtenir une bonne réticulation du liant dans le matelas y compris pour des liants nécessitant un contrôle précis de la température lors de la cuisson du matelas.

A cet effet, un objet de l'invention est un procédé de mesure à l'intérieur d'un matelas de fibres minérales et/ou végétales, et un liant, en déplacement avec au moins un convoyeur à bande transporteuse,
dans lequel le procédé utilise un système de mesure comprenant un capteur et un actionneur pour introduire le capteur dans le matelas, l'actionneur étant monté sur la bande transporteuse et configuré de façon à pouvoir déplacer le capteur entre une position rétractée et une position de mesure à l'intérieur du matelas, le procédé comprenant une introduction du capteur dans le matelas par l'actionneur sous l'effet du déplacement de la bande transporteuse.

Le procédé de mesure permet de connaître la température à l'intérieur du matelas (i.e. quelle que soit la position dans l'épaisseur), à tout instant du parcours du matelas dans l'étuve. Il est ainsi possible de vérifier de façon répétitive et même systématique, la température à l'intérieur du matelas le long de son parcours dans l'étuve. Il est notamment possible de vérifier si la température de réticulation a été atteinte et à quelle vitesse, dépassée de plus de X°C et combien de temps,...

Le procédé autorise l'optimisation du chauffage et du séchage du matelas, notamment lorsque l'épaisseur, la densité ou l'humidité du matelas change.

Suivant des modes particuliers de réalisation, le procédé inclut l'une ou plusieurs des étapes suivantes prise(s) isolément ou suivant toutes les combinaisons techniquement possibles :
- le procédé comprend en outre un retrait du capteur hors du matelas ;
- dans la position de mesure, le capteur est en saillie à l'extérieur de la bande transporteuse ;
- dans la position rétractée, le capteur est rétracté à l'intérieur de la bande transporteuse ;
- l'actionneur est autonome et passif ;
- l'actionneur comprend une masse d'actionnement dont le déplacement sous l'effet de la gravité et du déplacement de la bande transporteuse, déplace le capteur de la position rétractée à la position de mesure et/ou de la position de mesure à la position rétractée ;
- l'actionneur comprend un mécanisme de réglage de la profondeur de la position de mesure, autonome et passif ;
- l'actionneur comprend un mécanisme d'actionnement agissant sous l'effet de la déformation de la bande transporteuse en fin de bande ;
- la bande transporteuse est constituée d'éléments articulés, l'actionneur étant configuré pour utiliser le déplacement relatif des éléments articulés en bout de bande transporteuse pour déplacer le capteur ;
- le capteur est pourvu de et/ou forme par lui-même une masse d'actionnement déplaçant le capteur de la position rétractée à la position de mesure et/ou de la position de mesure à la position rétractée ;
- le capteur est sans fil, de préférence autonome et passif ;
- le capteur est un capteur de température ;
- le capteur est de type SAW ;
- le procédé comprend au moins une unité fixe de communication avec le capteur ;
- le système est configuré de telle sorte que le capteur puisse communiquer avec l'unité le long du trajet du convoyeur ;
- le procédé comprend une réticulation d'un liant présent dans le matelas par passage dans une étuve de réticulation, l'introduction du capteur étant réalisée dans l'étuve ou avant l'entrée dans l'étuve, et le retrait étant réalisée dans l'étuve ou après la sortie de l'étuve ;
- le procédé de mesure est utilisé dans un procédé de fabrication en continu de laine minérale.

L'invention a encore pour objet une ligne de fabrication d'un matelas de fibres minérales et/ou végétales, et un liant, comprenant au moins un convoyeur à bande transporteuse pour déplacer le matelas, et un système de mesure comprenant un capteur de mesure à l'intérieur du matelas et un actionneur pour introduire la capteur dans le matelas, l'actionneur étant monté sur la bande transporteuse et configuré de façon à pouvoir déplacer le capteur entre une position rétractée dans le convoyeur et une position de mesure à l'intérieur du matelas sous l'effet du déplacement de la bande transporteuse.

Suivant un mode particulier de réalisation, la ligne de fabrication précédente, comprend une étuve de réticulation d'un liant présent dans le matelas de fibres minérales, le convoyeur étant un convoyeur de transport du matelas dans l'étuve.

L'invention sera mieux comprise à la lecture de la description qui va suivre, fournie uniquement à titre d'exemple et faite en se référant aux dessins annexés sur lesquels :
- la figure 1 illustre une installation actuelle de fibrage d'un matelas en laine minérale ;
- la figure 2 est une représentation schématique en coupe d'une étuve de réticulation du matelas de la figure 1 ;
- la figure 3 donne une représentation schématique en coupe d'une partie de l'étuve de la figure 2, montrant un système de mesure à l'intérieur du matelas traversant l'étuve, le système de mesure étant monté sur la bande transporteuse du convoyeur du matelas, la figure 3 illustrant schématiquement le principe de fonctionnement du capteur ;
- les figures 4 à 4quater illustrent schématiquement un exemple de système de mesure utilisant le déplacement de la bande transporteuse du convoyeur pour déplacer le capteur de mesure ; et
- la figure 5 est un vue en perspective de dessous d'une variante de réalisation pour l'actionneur.

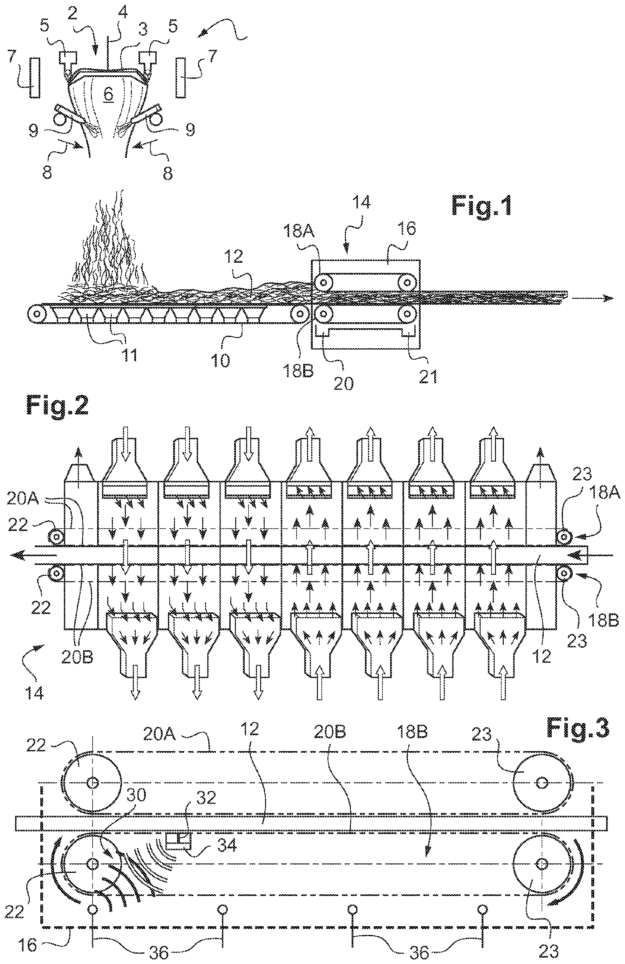

La figure 1 représente les premières étapes d'une ligne de production d'un matelas continu de fibres minérales, plus particulièrement à base de laine de verre, étant entendu que la ligne est de tout type adapté pour la production de produits à base de fibres minérales et éventuellement végétales.

A titre d'exemple pour la laine de verre, la ligne comporte une unité de fibrage 1, par exemple conforme au procédé de fibrage par centrifugation interne. L'unité de fibrage comporte une hotte (non représentée sur la figure 1) surmontée d'au moins un centrifugeur 2. Chaque centrifugeur comprend un panier (non représenté sur la figure 1) pour la récupération d'un filet de verre de fibrage préalablement fondu et une pièce 3 en forme d'assiette dont la paroi périphérique est munie d'un grand nombre d'orifices. En fonctionnement, le verre fondu, amené en un filet 4 depuis un four de fusion (non représenté) et d'abord récupéré dans le panier du centrifugeur, s'échappe par les orifices de l'assiette 3 sous la forme d'une multitude de filaments entraînés en rotation. Le centrifugeur 2 est par ailleurs entouré par un brûleur annulaire 5 qui crée à la périphérie de la paroi du centrifugeur un courant gazeux à grande vitesse et à température suffisamment élevée pour étirer les filaments de verre en fibres sous la forme d'un voile 6.

Des moyens de chauffage 7 par exemple du type inducteurs servent à maintenir le verre et le centrifugeur à la bonne température. Le voile 6 est refermé par un courant gazeux d'air introduit sous pression, schématisé par les flèches 8. Le voile 6 est entouré par un dispositif de pulvérisation de l'encollage contenant un liant thermodurcissable en solution aqueuse, dont seuls deux éléments 9 sont représentés sur la figure 1.

Il s'agit par exemple d'un liant phénolique ou un liant alternatif à faible teneur en formaldéhyde, liants parfois qualifiés de « liants verts », notamment lorsqu'ils sont au moins partiellement issus d'une base de matière première renouvelable, en particulier végétale, notamment du type à base de sucres hydrogénés ou non.

Le fond de la hotte de fibrage est constitué par un dispositif de réception des fibres comprenant un convoyeur incorporant une bande sans fin 10 perméable aux gaz et à l'eau, à l'intérieur de laquelle sont disposés des caissons d'aspiration 11 des gaz tels que l'air, les fumées et les compositions aqueuses excédentaires issues du processus de fibrage précédemment décrit. Il se forme ainsi sur la bande 10 du convoyeur un matelas 12 de fibres de laine de verre mélangées intimement avec la composition d'encollage. Le matelas 12 est conduit par le convoyeur 10 jusqu'à une étuve 14 de réticulation du liant thermodurcissable.

Comme représenté sur les figures 1 et 2, cette étuve 14 est entourée par une enceinte 16 fermée (hormis autour du matelas à l'entrée et à la sortie) délimitant des sas d'entrée et de sortie et une série de caissons séparés les uns des autres par des parois et individuellement alimentés en air chaud par des brûleurs mis en circulation par des ventilateurs (non représentés sur les figures 1 et 2). L'enceinte est traversée par deux convoyeurs 18A, 18B de transport et de calibrage du matelas 12. Il s'agit d'un convoyeur supérieur 18A et d'un convoyeur inférieur 18B en regard l'un de l'autre. La distance entre les convoyeurs 18A, 18B est réglable, de façon à calibrer l'épaisseur du matelas 12.

Ces convoyeurs 18A, 18B comprennent chacun une bande transporteuse sans fin 20A, 20B constituée chacune par une succession de palettes en forme de grille, articulées entre elles, au moins un moteur placé au sol ou sur un bâti approprié (20, 21 sur la figure 1), et des rouleaux d'extrémité 22, 23 reliés au(x) moteur(s) 20, 21 pour un entraînement des bandes 20A, 20B. Les palettes sont plus généralement des plaques métalliques perforées, ou plus généralement encore des éléments de convoyage perméables aux gaz et assemblés en une bande sans fin.

Tout en assurant le passage des gaz chauds favorisant la prise rapide du liant, les convoyeurs 18A, 18B compriment le matelas 12 pour lui donner l'épaisseur souhaitée. A titre d'exemple, pour un produit fini, celle-ci est typiquement comprise entre 10 et 450 mm, la densité de la couche de laine de verre étant par exemple comprise entre 5 et 250 kg/m3. On distingue ainsi par exemple les produits dits de faible densité, pour lesquels la masse volumique varie entre 5 et 15kg/m3 et les produits dits de moyenne densité entre 15 et 40kg/m3 et les produits de forte densité au-delà.

Les sas d'entrée et de sortie débouchent sur des hottes de sortie des fumées (dont le sens d'évacuation est représenté sur la figure 2 par des flèches), ces hottes étant reliées à un circuit dédié de traitement desdites fumées (non représenté sur les figures).

Sur les figures, la circulation de l'air dans l'étuve est représentée par des flèches.

A titre d'exemple, dans les premiers caissons, l'air chaud est introduit par le bas de l'étuve et évacué par le haut, après traversée du matelas. L'utilisation d'une pluralité de caissons permet la montée en température progressive du matelas de fibres jusqu'à une température supérieure ou égale à la température de réticulation du liant présent sur les fibres du matelas.

Dans les caissons suivants, l'air chaud est cette fois introduit par le haut de l'étuve et évacué par le bas.

Les fumées supplémentaires générées dans les caissons sont finalement évacuées dans le sas de sortie ou l'entrée, via les hottes.

D'une manière générale, la température de l'air chaud soufflé dans l'étuve dans les caissons 24-30 est supérieure à la température de réticulation du liant (aussi appelée température de « durcissement »), par exemple un air chaud entre 180°C et 300°C.

Plus particulièrement selon l'invention, comme illustré sur la figure 3, l'un 20B des convoyeurs 20A, 20B traversant l'étuve, plus particulièrement sa bande transporteuse, est équipé d'un système 30 de mesure de la température à coeur (plus généralement à l'intérieur) du matelas 12.

A titre de variante, il s'agit d'un système de mesure d'une autre caractéristique de tout type adapté, telle que l'humidité.

Le système 30 comprend un capteur 32 de mesure et un actionneur 34 du capteur 32 pour le déplacer entre une position de repos et une position de mesure.

La position de repos est une position rétractée à l'intérieur du convoyeur 18B, plus précisément à l'intérieur de la bande transporteuse 20B et la position de mesure est une position en saillie à l'extérieur de la bande transporteuse 20B, plus particulièrement à l'intérieur du matelas 12 présent sur ou sous le bande transporteuse 20A, 20B.

A titre de remarque, le système de mesure 30 est monté sur l'un ou l'autre de la bande transporteuse 20A du convoyeur supérieur 18A et de la bande transporteuse 20B du convoyeur inférieur 18B, ou par exemple sur chacun dans le cas où il y aurait plusieurs systèmes de mesure.

Le capteur 32 présente la particularité d'être de type passif, par exemple de type SAW (« surface acoustic wave »). Ces capteurs ne nécessitent pas d'être reliés par des fils ou même d'être alimentés électriquement par une batterie. Ils fournissent l'information « température » ou plus généralement l'information « mesure » par modulation du champ électromagnétique envoyé par une antenne, laquelle interprète cette modification du champ. Ces capteurs ne nécessitent pas donc d'être solidaires du bâti mais simplement à distance suffisante de l'unité de communication, qui est, elle, par exemple solidaire du bâti, à l'intérieur de l'enceinte 16.

De préférence, le capteur 32 est introduit à une profondeur correspondant à la moitié de l'épaisseur du matelas 12, pour une température à coeur.

En variante cependant, la profondeur à l'intérieur du matelas 12 est par exemple de toute valeur adaptée.

Dans l'exemple schématique illustré sur la figure 3, l'introduction du capteur 32 dans le matelas 12 est réalisée après son entrée dans l'étuve 14 et le capteur 32 retiré avant sa sortie. D'une manière générale cependant, le capteur 32 est inséré avant ou après l'entrée dans l'étuve, et rétracté avant ou après sa sortie de l'étuve 14, c'est-à-dire que le capteur 32 est inséré dans le matelas 12 au moins à un moment de sa traversée de l'étuve 14. En variante cependant, le capteur pourrait être introduit par exemple immédiatement après la sortie du matelas de l'étuve 14.

L'actionneur 34 du capteur 32 est autonome et passif, comme expliqué plus détail ci-après. On entend par autonome qu'il ne nécessite ni alimentation électrique à distance ni alimentation d'aucune sorte, et on entend par passif qu'il actionne le déplacement du capteur sous l'effet d'un élément extérieur, à savoir ici le déplacement de la bande transporteuse. Le déplacement de la bande transporteuse a deux effets : un effet de déplacement du capteur, plus particulièrement un effet de retournement du capteur en bout de bande, permettant d'utiliser l'effet de la gravité pour l'actionnement passif du mécanisme, comme expliqué plus en détail ci-après, et un effet de déformation de la bande transporteuse en bout de bande, laquelle déformation peut également être utilisée de façon passif par l'actionneur pour déplacer le capteur.

La figure 3 est un schéma de principe de fonctionnement global du système.

L'actionneur 34 est monté sur la bande transporteuse 20B du convoyeur 18B (i.e. la partie de convoyage), et de ce fait solidaire de la bande transporteuse 20B, i.e. solidaire du déplacement de la bande transporteuse.

Le capteur 32 communique, le long de son trajet dans l'étuve 14, avec les différentes antennes successives 36 placées sur son trajet, à l'intérieur de l'étuve 14.

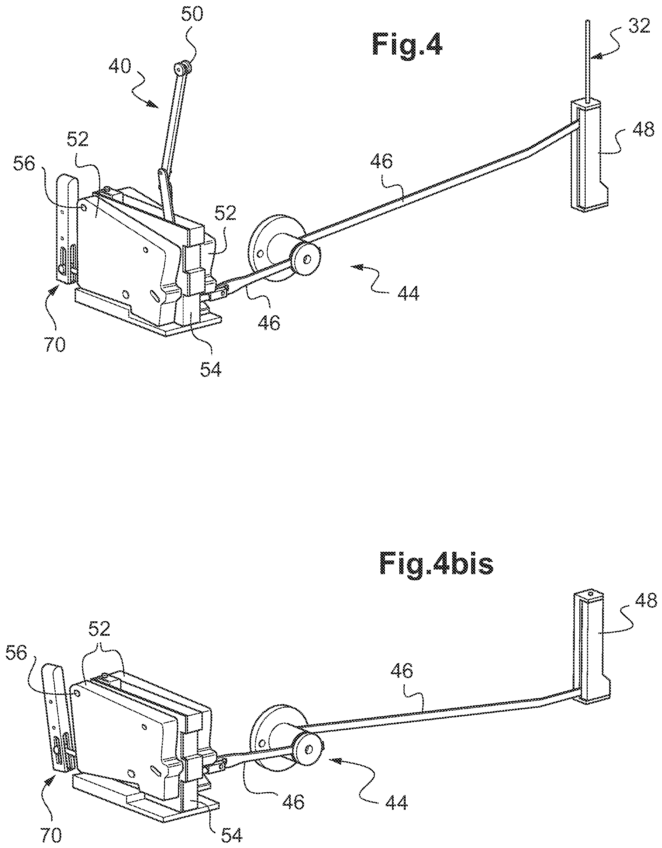

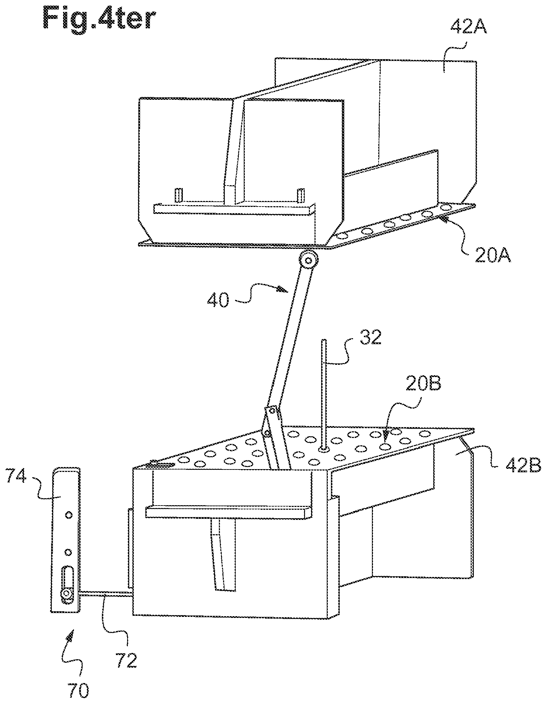

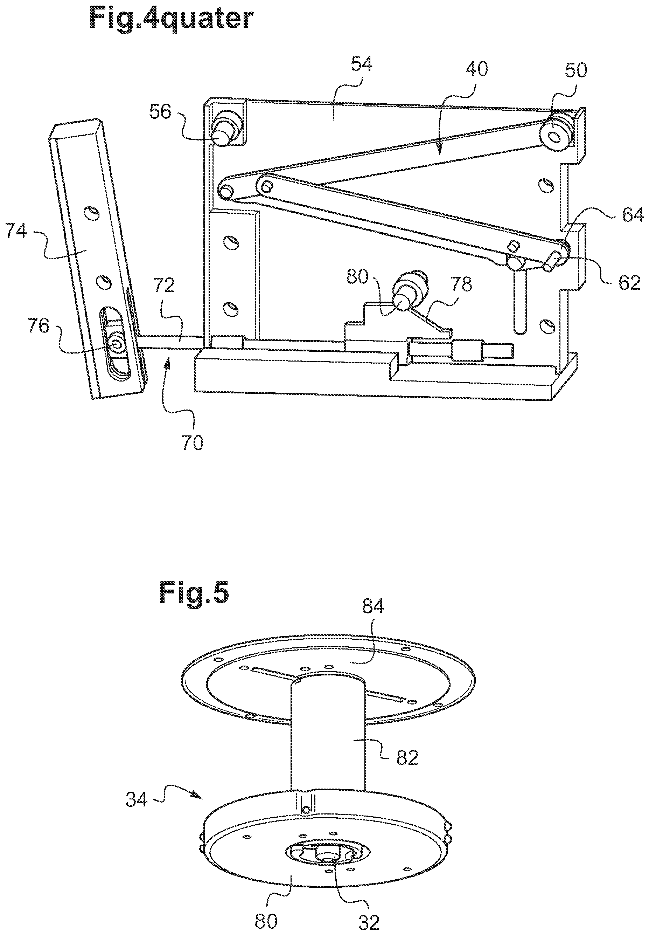

Les figures 4 à 5 illustrent des exemples possibles d'actionneurs passifs et autonomes.

Sur les figures 4 à 4quater, l'actionneur 34 possède un bras articulé 40 de mesure de l'épaisseur du matelas 12, mobile entre une position rétractée (voir figures 4 bis et 4quater) à l'intérieur de la bande transporteuse 20B, i.e. du convoyeur 18B, et une position de contact avec le convoyeur opposé 18A, plus particulièrement avec la bande transporteuse opposée 20A (voir figures 4 et 4ter). A noter que sur la figure 4ter, seule une palette 42A, 42B de chaque bande transporteuse 20A, 20B a été représentée. Des espaces sont ménagés dans la palette 42B pour le déplacement du bras articulé 40 et du capteur 32 à travers cette dernière.

Un mécanisme 44 d'entraînement à deux bielles 46 asservit le déplacement vertical du capteur 32 dans son logement 48 à un déplacement moitié moindre verticalement à celui de l'extrémité 50 du bras 40. Lorsque le bras 40 est en contact avec la bande transporteuse opposée 20A, l'actionneur déplace ainsi l'extrémité du capteur 32 à mi-distance entre les deux convoyeurs 18A, 18B, i.e. à coeur du matelas. En variante cependant, cet asservissement du déplacement du capteur 32 à celui du bras 40 est de tout autre type adapté.

Le déplacement du bras 40 de sa position rétractée à sa position sortie de contact avec le convoyeur opposé est obtenu par deux plaques 52 formant masses d'actionnement, montées en rotation sur le boîtier 54 logent le bras 40 (la figure 4ter montre l'intérieur du boîtier 54). Les deux plaques 52 sont montées en rotation sur l'axe 56 (figure 4, 4bis et 4quater) et reçoivent en liaison pivot glissante l'axe 62 solidaire du bras 40 (voir notamment figure 4quater). La rotation des plaques 52 vers le bas autour de leur axe 56 appuie sur l'axe 62 monté sur l'extrémité proximale 64 du bras articulé 40 et sort le bras 40. En variante, il s'agit de masses d'actionnement de tout type adapté, l'actionneur étant configuré pour déplacer le bras 40 et le capteur 32 par au moins une masse d'actionnement, sous l'effet de la gravité. Plus généralement encore, l'actionneur 34 est dépourvu de bras articulé de mesure de l'épaisseur, comme nous le verrons dans le mode de réalisation de la figure 5.

Comme visible plus en détail sur la figure 4quater, l'actionneur 32 est également pourvu d'un mécanisme 70 de rétraction du bras 40 et du capteur 32 à l'intérieur de la bande transporteuse 20B, l'actionneur 70 étant configuré pour contrer l'action des masses d'actionnement 52.

Le mécanisme 70 comprend une bielle 72 montée coulissante dans un logement 74 fixée sur la palette 42B adjacente de la bande transporteuse 20B. Lorsque le système de mesure 40 arrive en bout de bande transporteuse 20B, la palette en amont et la palette 42B portant le capteur 32 commencent une rotation l'une par rapport à l'autre, si bien que l'extrémité 76 (figure 4 quater) de la bielle 72 engagée dans le logement 74 se rapproche du boîtier 54. Une surface de came 78 solidaire de la bielle 72 déplace alors l'axe 80, lui-même monté purement pivotant dans les plaques d'actionnement 52. Les plaques 52 sont alors remontées jusqu'à leur position relevée par rotation autour de leur axe 56, ce qui actionne le bras 40 et le capteur 32 jusqu'à leur position rétractée à l'intérieur de la bande transporteuse 20B par l'intermédiaire de l'axe 62. En variante, le mécanisme 70 est de tout type adapté. D'une manière générale, l'actionneur 34 est configuré pour utiliser la déformation de la bande transporteuse en bout de bande afin de déplacer le capteur 32. L'actionneur déplace le capteur 32 de sa position rétractée à sa position de mesure et de sa position de mesure à sa position rétractée de façon autonome et passive.

La figure 5 représente une variante de réalisation de l'actionneur 34, dépourvu de mécanisme de mesure de l'épaisseur du matelas 12 et utilisant le seul effet de la gravité pour déplacer le capteur 32 dans les deux sens.

L'actionneur comprend une masse d'actionnement 80 en forme de disque montée mobile en translation sur un cylindre 82, entre une première position basse (figure 5) et une deuxième position basse. D'une manière générale, il s'agit d'une masse de tout type adapté et notamment de toute taille, densité et forme adaptées.

Lors de la bascule du système de mesure en bout de bande, la masse se déplace, sous l'effet de la gravité, de sa première position basse, éloignée du socle de support 84 à une nouvelle position basse, plus proche du socle 84.

La masse est par exemple reliée par des câbles au capteur 32, lui-même monté mobile en translation dans cet exemple à l'intérieur du cylindre 82. Le mouvement de la masse 80 commande le mouvement du capteur 32 entre sa position de repos et sa position de mesure.

En variante, la masse 80 ne commande que la sortie du capteur 32 vers sa position de mesure, la rentrée du capteur 32 étant commandée par sa propre masse (par exemple dans le cas d'un montage sur le convoyeur supérieur 18A), ou inversement.

En variante, encore, le capteur 32 est alourdi par une masse. De cette façon, si le capteur 32 est sur le convoyeur supérieur 18A, il entre dans le matelas et/ou sort du matelas uniquement sous l'effet de la gravité agissant sur le capteur.

D'une manière générale, il s'agit d'un actionneur passif et autonome.

Le système de mesure 30 selon l'invention présente l'avantage de permettre de mesurer une caractéristique telle la température à coeur du matériau, à l'intérieur d'une étuve de réticulation, et ce tout le long du trajet du capteur dans l'étuve. Qui plus est, la mesure est continuellement déterminable par l'unité de communication.

Le pilotage de l'étuve, et notamment le séchage et le chauffage peuvent prendre en compte la température mesurée et un opérateur peut commander une action corrective de façon manuelle ou bien un système de contrôle peut commander une action corrective en fonction de consignes prédéterminées.

On conçoit tout l'intérêt d'un tel système dans un procédé de fabrication de laine minérale.

## Revendications

1. Procédé de mesure à l'intérieur d'un matelas comprenant des fibres minérales et/ou végétales, et un liant, en déplacement avec au moins un convoyeur (18A, 18B) à bande transporteuse (20A, 20B),
dans lequel le procédé utilise un système de mesure (30) comprenant un capteur (32) et un actionneur (34) pour introduire le capteur dans le matelas (12), l'actionneur étant monté sur la bande transporteuse et configuré de façon à pouvoir déplacer le capteur entre une position rétractée et une position de mesure à l'intérieur du matelas, le procédé comprenant une introduction du capteur dans le matelas par l'actionneur sous l'effet du déplacement de la bande transporteuse.

2. Procédé selon la revendication 1, comprenant en outre un retrait du capteur hors du matelas.

3. Procédé selon la revendication 1 ou 2, dans lequel, dans la position de mesure, le capteur est en saillie à l'extérieur de la bande transporteuse.

4. Procédé selon l'une quelconque des revendications précédentes, dans lequel, dans la position rétractée, le capteur est rétracté à l'intérieur de la bande transporteuse.

5. Procédé selon l'une quelconque des revendications précédentes, dans lequel l'actionneur est autonome et passif.

6. Procédé selon la revendication précédente, dans lequel l'actionneur comprend une masse d'actionnement (52, 80) dont le déplacement sous l'effet de la gravité et du déplacement de la bande transporteuse (20B), déplace le capteur (32) de la position rétractée à la position de mesure et/ou de la position de mesure à la position rétractée.

7. Procédé selon l'une quelconque des revendications précédentes, dans lequel l'actionneur comprend un mécanisme (40) de réglage de la profondeur de la position de mesure, autonome et passif.

8. Procédé selon l'une quelconque des revendications 5 à 7, dans lequel l'actionneur comprend un mécanisme d'actionnement agissant sous l'effet de la déformation de la bande transporteuse en fin de bande.

9. Procédé selon la revendication précédente, dans lequel la bande transporteuse est constituée d'éléments articulés, l'actionneur étant configuré pour utiliser le déplacement relatif des éléments articulés en bout de bande transporteuse pour déplacer le capteur.

10. Procédé selon l'une quelconque des revendications 5 à 9, dans lequel le capteur est pourvu et/ou forme par lui-même masse d'actionnement déplaçant le capteur (32) de la position rétractée à la position de mesure et/ou de la position de mesure à la position rétractée.

11. Procédé selon l'une quelconque des revendications précédentes, dans lequel le capteur est sans fil, de préférence autonome et passif.

12. Procédé selon l'une quelconque des revendications précédentes, dans lequel le capteur est un capteur de température.

13. Procédé selon l'une quelconque des revendications précédentes, dans lequel le capteur est de type Surface Acoustic Wave (SAW).

14. Procédé selon l'une quelconque des revendications précédentes, comprenant au moins une unité fixe de communication avec le capteur.

15. Procédé selon la revendication précédente, dans lequel le système est configuré de telle sorte que le capteur puisse communiquer avec l'unité le long du trajet du convoyeur.

16. Procédé selon l'une quelconque des revendications précédentes, comprenant une réticulation d'un liant présent dans le matelas par passage dans une étuve de réticulation, l'introduction du capteur étant réalisée dans l'étuve ou avant l'entrée dans l'étuve, et le retrait étant réalisée dans l'étuve ou après la sortie de l'étuve.

17. Procédé selon l'une quelconque des revendications précédentes, dans un procédé de fabrication en continu de laine minérale.

18. Ligne de fabrication d'un matelas (12) de fibres minérales et/ou végétales, et un liant, comprenant au moins un convoyeur (18A, 18B) à bande transporteuse (20A, 20B) pour déplacer le matelas, et un système de mesure comprenant un capteur (32) de mesure à l'intérieur du matelas et un actionneur (34) pour introduire la capteur dans le matelas, l'actionneur étant monté sur la bande transporteuse et configuré de façon à pouvoir déplacer le capteur entre une position rétractée dans le convoyeur et une position de mesure à l'intérieur du matelas sous l'effet du déplacement de la bande transporteuse.

19. Ligne selon la revendication précédente, comprenant une étuve (14) de réticulation d'un liant présent dans le matelas de fibres minérales, le convoyeur étant un convoyeur de transport du matelas dans l'étuve.

## Patentansprüche

1. Verfahren zur Messung innerhalb einer Matte, umfassend Mineral- und/oder Pflanzenfasern und ein Bindemittel, in Verlagerung mit mindestens einem Förderer (18A, 18B) mit Förderband (20A, 20B),
wobei bei dem Verfahren ein Messsystem (30) verwendet wird, das einen Sensor (32) und ein Stellglied (34) umfasst, um den Sensor in die Matte (12) einzuführen, wobei das Stellglied auf dem Förderband montiert und konfiguriert ist, um den Sensor zwischen einer eingefahrenen Position und einer Messposition innerhalb der Matte verlagern zu können, wobei das Verfahren ein Einführen des Sensors in die Matte durch das Stellglied unter der Wirkung der Verlagerung des Förderbandes umfasst.

2. Verfahren nach Anspruch 1, ferner umfassend ein Einfahren des Sensors aus der Matte.

3. Verfahren nach Anspruch 1 oder 2, wobei der Sensor in der Messposition über das Äußere des Förderbandes hinausragt.

4. Verfahren nach einem der vorstehenden Ansprüche, wobei der Sensor in der eingefahrenen Position in das Innere des Förderbandes eingefahren ist.

5. Verfahren nach einem der vorstehenden Ansprüche, wobei das Stellglied autonom und passiv ist.

6. Verfahren nach dem vorstehenden Anspruch, wobei das Stellglied eine Betätigungsmasse (52, 80) umfasst, deren Verlagerung unter der Wirkung der Schwerkraft und der Verlagerung des Förderbandes (20B) den Sensor (32) von der eingefahrenen Position in die Messposition und/oder von der Messposition in die eingefahrene Position verlagert.

7. Verfahren nach einem der vorstehenden Ansprüche, wobei das Stellglied einen Mechanismus (40) zum Einstellen der Tiefe der Messposition, autonom und passiv, umfasst.

8. Verfahren nach einem der Ansprüche 5 bis 7, wobei das Stellglied einen Betätigungsmechanismus umfasst, der unter der Wirkung der Verformung des Förderbandes am Ende des Bandes wirkt.

9. Verfahren nach dem vorstehenden Anspruch, wobei das Förderband aus gelenkigen Elementen besteht, wobei das Stellglied konfiguriert ist, um die relative Verlagerung der gelenkigen Elemente am Ende des Förderbandes zum Verlagern des Sensors zu nutzen.

10. Verfahren nach einem der Ansprüche 5 bis 9, wobei der Sensor versehen ist mit einer und/oder selbst eine Betätigungsmasse bildet, die den Sensor (32) von der eingefahrenen Position in die Messposition und/oder von der Messposition in die eingefahrene Position verlagert.

11. Verfahren nach einem der vorstehenden Ansprüche, wobei der Sensor drahtlos, vorzugsweise autonom und passiv ist.

12. Verfahren nach einem der vorstehenden Ansprüche, wobei der Sensor ein Temperatursensor ist.

13. Verfahren nach einem der vorstehenden Ansprüche, wobei der Sensor vom Typ Akustikoberflächenwelle (SAW, Surface Acoustic Wave) ist.

14. Verfahren nach einem der vorstehenden Ansprüche, umfassend mindestens eine feste Kommunikationseinheit mit dem Sensor.

15. Verfahren nach dem vorstehenden Anspruch, wobei das System so konfiguriert ist, dass der Sensor mit der Einheit entlang des Weges des Förderers kommunizieren kann.

16. Verfahren nach einem der vorstehenden Ansprüche, umfassend ein Vernetzen eines in der Matte vorhandenen Bindemittels durch Durchlaufen eines Vernetzungsofens, wobei das Einführen des Sensors im Ofen oder vor dem Eintritt in den Ofen durchgeführt wird und das Einfahren im Ofen oder nach dem Verlassen des Ofens durchgeführt wird.

17. Verfahren nach einem der vorstehenden Ansprüche in einem kontinuierlichen Mineralwollherstellungsverfahren.

18. Linie zur Herstellung einer Matte (12) aus Mineral- und/oder Pflanzenfasern und einem Bindemittel mit mindestens einem Förderer (18A, 18B) mit Förderband (20A, 20B) zum Verlagern der Matte und einem Messsystem, das einen Messsensor (32) zum Messen innerhalb der Matte und ein Stellglied (34) umfasst, um den Sensor in die Matte einzuführen, wobei das Stellglied auf dem Förderband montiert und konfiguriert ist, um den Sensor zwischen einer eingefahrenen Position in dem Förderer und einer Messposition innerhalb der Matte unter der Wirkung der Verlagerung des Förderbandes verlagern zu können.

19. Linie nach dem vorstehenden Anspruch, umfassend einen Ofen (14) zum Vernetzen eines in der Mineralfasermatte vorhandenen Bindemittels, wobei der Förderer ein Förderer zum Transportieren der Matte in den Ofen ist.

## Claims

1. Method for measuring inside a blanket comprising mineral and/or plant fibres , and a binder, being moved by at least one conveyor (18A, 18B) with a conveyor belt (20A, 20B),
wherein the method uses a measuring system (30) comprising a sensor (32) and an actuator (34) for introducing the sensor into the blanket (12), the actuator being mounted on the conveyor belt and configured to be able to move the sensor between a retracted position and a measuring position inside the blanket, the method comprising introduction of the sensor into the blanket by the actuator under the effect of the movement of the conveyor belt.

2. Method according to Claim 1, further comprising removal of the sensor from the blanket.

3. Method according to Claim 1 or 2, wherein, in the measuring position, the sensor projects out of the conveyor belt.

4. Method according to any one of the preceding claims, wherein, in the retracted position, the sensor is retracted inside the conveyor belt.

5. Method according to any one of the preceding claims, wherein the actuator is autonomous and passive.

6. Method according to the preceding claim, wherein the actuator comprises an actuating mass (52, 80), the movement of which under the effect of gravity and of the movement of the conveyor belt (20B) moves the sensor (32) from the retracted position to the measuring position and/or from the measuring position to the retracted position.

7. Method according to any one of the preceding claims, wherein the actuator comprises a mechanism (40) for adjusting the depth of the measuring position, said mechanism being autonomous and passive.

8. Method according to any one of Claims 5 to 7, wherein the actuator comprises an actuating mechanism that acts under the effect of the deformation of the conveyor belt at the end of the belt.

9. Method according to the preceding claim, wherein the conveyor belt is formed by articulated elements, the actuator being configured to use the relative movement of the articulated elements at the end of the conveyor belt to move the sensor.

10. Method according to any one of Claims 5 to 9, wherein the sensor is provided with and/or itself forms an actuating mass that moves the sensor (32) from the retracted position to the measuring position and/or from the measuring position to the retracted position.

11. Method according to any one of the preceding claims, wherein the sensor is wireless, and preferably autonomous and passive.

12. Method according to any one of the preceding claims, wherein the sensor is a temperature sensor.

13. Method according to any one of the preceding claims, wherein the sensor is of the Surface Acoustic Wave (SAW) type.

14. Method according to any one of the preceding claims, comprising at least one fixed unit for communication with the sensor.

15. Method according to the preceding claim, wherein the system is configured such that the sensor can communicate with the unit along the path of the conveyor.

16. Method according to any one of the preceding claims, comprising crosslinking of a binder present in the blanket by passage through a crosslinking oven, the sensor being introduced in the oven or before entering the oven, and being removed in the oven or after exiting the oven.

17. Method according to any one of the preceding claims, in a continuous method for producing mineral wool.

18. Line for manufacturing a blanket (12) of mineral and/or plant fibres and a binder, comprising at least one conveyor (18A, 18B) with a conveyor belt (20A, 20B) for moving the blanket, and a measuring system comprising a sensor (32) for measuring inside the blanket and an actuator (34) for introducing the sensor into the blanket, the actuator being mounted on the conveyor belt and configured to be able to move the sensor between a retracted position in the conveyor and a measuring position inside the blanket under the effect of the movement of the conveyor belt.

19. Line according to the preceding claim, comprising an oven (14) for crosslinking a binder present in the blanket of mineral fibres, the conveyor being a conveyor for transporting the blanket through the oven.
